# EUROPÄISCHE PATENTSCHRIFT

(11) **EP 0 826 966 B1**
(45) Veröffentlichungstag und Bekanntmachung des Hinweises auf die Patenterteilung: **01.02.2006**
(21) Anmeldenummer: 97112828.5
(22) Anmeldetag: 25.07.1997
(51) Int. Cl.: G01N 33/96, A61K 35/14

(54) **Methode zur Stabilisierung von Plättchen**
Method for stabilizing platelets
Méthode de stabilisation de plaquettes

(30) Priorität: 24.08.1996 DE 19634313
(43) Veröffentlichungstag der Anmeldung: 04.03.1998
(73) Patentinhaber: Dade Behring Marburg GmbH, 35001 Marburg (DE)
(72) Erfinder: Braun, Konrad, 35085 Ebsdorfergrund (DE)

(56) Entgegenhaltungen:
- EP-A- 0 433 927
- WO-A-93/23997
- WO-A-96/13158
- US-A- 4 405 719
- BODE A P ET AL: "THE USE OF INHIBITORS OF PLATELET ACTIVATION OR PROTEASE ACTIVITY IN PLATELET CONCENTRATES STORED FOR TRANSFUSION" BLOOD CELLS, Bd. 18, Nr. 3, 1. Januar 1992, Seiten 361-380, XP000566738

## Beschreibung

Die vorliegende Erfindung betrifft Präparationen funktionell aktiver Plättchen und ein Verfahren zur Stabilisierung solcher funktionell aktiven Plättchen durch Gefriertrocknung.

Zugabe von inhibitoren der Plättchenfunktion sowie nachfolgender Funktionell aktive Plättchen sind in diagnostischer und therapeutischer Hinsicht von Nutzen. Ein Aspekt der Nutzung eines Präparats funktionell aktiver Plättchen ist zum Beispiel die Verwendung als Kontrollmaterial für die Plättchenfunktionsdiagnostik. In dieser Diagnostik spielt die Aggregometrie die hervorragende Rolle. Gemessen wird dabei die Reaktion von frischen Plättchen, die in der Regel als plättchenreiches Plasma vorliegen, auf verschiedene Induktoren, vor allem ADP, Adrenalin, Arachidonsäure, Kollagen und Thrombin. Die Messung der Reaktion erfolgt üblicherweise nach den bekannten turbidimetrischen Methoden. Die Induktoren (außer Adrenalin) bewirken zunächst einen Formenwandel der Zellen, der an einer vorübergehenden Erhöhung der Extinktion erkennbar ist. Anschließend erfolgt die Aggregation. Oft entstehen biphasische Aggregationskurven. Die zweite Phase ist eng mit der Freisetzungsreaktion und mit Prostaglandinsynthese verknüpft. Arachidonsäure und Kollagen induzieren nur monophasische Aggregationskurven als Ausdruck irreversibler Aggregation.

Aus Gründen der Rationalisierung und Präzision ist die Untersuchung der Plättchenfunktion heute weitgehend automatisiert. Zur Beurteilung der Ergebnisse ist eine Qualitätskontrollstrategie unbedingt notwendig. Die einzige Möglichkeit zur Kontrolle dieser Diagnostik besteht bis heute in der Verwendung eines Pools aus Proben 'normaler' Spender (Abernathy et al. (1978) Throm.Haemostas. 39, S. 246) Dies ist für das Labor ein nur mit großem Aufwand herzustellendes Kontrollmaterial. Eine stabile und funktionell aktive Plättchenpräparation mit definierten und konstanten Eigenschaften zur Kontrolle der Plättchenfunktion ist wünschenswert, steht aber bislang nicht zur Verfügung.

Ein weiterer Aspekt des Einsatzes stabiler, funktionell aktiver Plättchen ist die Verwendung in diagnostischen Testverfahren, in denen Plättchen eines der einzusetzenden Reagenzien ist. Ein Beispiel hierfür sind Teste zur Erkennung der heparinassoziierten Thrombozytopenie (HAT). Hierbei handelt es sich um eine seltene (ca. 5 %), aber schwere Komplikation der antithrombotischen Therapie mit Heparinen. Unterschieden werden nicht-immunologische Formen (HAT I) von immunologisch bedingten Formen (HAT II). Im Gegensatz zu anderen medikamentös induzierten Thrombozytopenien, die in der Regel Blutungskomplikationen hervorrufen, kommt es bei der HAT zu thromboembolischen Komplikationen, die bis zu Verschlüssen großer Gefäße reichen können. Zur Früherkennung einer Typ II HAT dienen verschiedene diagnostischen Ansätze. Neben der Zählung von Plättchen, die ein erster Suchtest ist, und dem Serotonin-Freisetzungstest als Referenzmethode kommt vor allem ein Test auf Heparin-induzierte Plättchenaggregation (HIPA) in Frage, der in Sensitivität und Spezifität der Referenzmethode nahe kommt (Greinacher et al. (1991) Thromb.Haemostas. 66(6),734,1991 Greinacher et al.(1994) Transfusion 34 , S. 381). Hierbei wird untersucht, ob sich Thrombozyten gesunder Spender unter geeigneten Bedingungen (niedrige Heparinkonzentration) durch Patientenplasma zur Aggregation bringen lassen. Die Herstellung der Thrombozyten, die das Poolen von plättchenreichem Plasma mehrerer gesunder Spender erfordert, ist für das Labor sehr aufwendig und steht bisher die Einführung des Testes in die Laborroutine im Wege. Eine Präparation stabiler Thrombozyten, die noch insoweit funktionell aktiv sind, daß sie sich unter geeigneten Bedingungen aggregieren lassen, würde die Durchführung dieses Testes erheblich vereinfachen und beschleunigen.

Therapeutisch lassen sich Plättchenkonzentrate zur Behandlung von Plättchenfunktionsstörungen verschiedener Genese einsetzen. Bei Plättchenkonzentrationen von < 70 000 pro Mikroliter spricht man von Thrombozytopenien. Thrombozytopenien beruhen entweder auf einer unzureichenden Produktion von Plättchen, einem beschleunigten Abbau dieser Zellen oder einer nicht normalen Verteilung (Colman et al. (1987) Haemostasis and Thrombosis (J.B. Lipincott Co.) 2nd Ed., Kapitel 28 ). Eine unzureichende Plättchenfunktion kann grundsätzlich genetisch bedingt (z.B.: Glanzmanns Thrombasthenie oder Bernard-Soulier-Syndrom) oder erworben sein (z.B. Versagen des Knochenmarks bei malignen Erkrankungen, Chemotherapie, Disseminierte Intravasale Gerinnung). Eine Reihe von Drogen, Medikamenten und ionisierenden Strahlen können zu erworbenen Thrombozytopenien führen.

Patienten, die an einer Thrombozytopenie leiden, haben eine Blutungstendenz ähnlich wie Hämophile. Die Blutungen erfolgen in der Regel aus Kapillargefäßen; typisch sind z.B. kleinere Einblutungen in die Schleimhäute (Petechien). Normalerweise werden kleinere Verletzungen der Kapillargefäßwand durch agglutinierende Plättchen verschlossen.

Heutzutage werden Patienten mit niedrigen Plättchenzahlen mit Infusion von Plättchenkonzentraten behandelt. Diese Konzentrate enthalten typischerweise 6 x 10¹⁰ Plättchen in ca. 50 mL Plasma. Sie werden hergestellt durch stufenweise Zentrifugation von antikoaguliertem Blut und Wiederaufnahme des Plättchen-Niederschlags in Plasma. Alternativ können Plättchenkonzentrate durch eine Aphereseapparatur hergestellt werden, durch die die Plättchen direkt aus Blut abgetrennt werden. Plättchenkonzentrate sind unter geeigneten Bedingungen (Raumtemperatur) bis zu 7 Tage haltbar. Während der Lagerungszeit müssen die Beutel, die das Konzentrat enthalten, permanent bewegt werden. Eine längere Haltbarkeit von Plättchenkonzentraten ist wünschenwert, konnte bisher aber nicht erreicht werden.

Eine erste mögliche Strategie zur Erreichung einer höheren Haltbarkeit von Plättchenkonzentraten zu diagnostischen oder therapeutischen Zwecken besteht in der Ausschaltung bestimmter Aktivierungsmechanismen der Plättchen. Dies soll gewährleisten, daß die Plättchen nicht bereits vorzeitig durch den Prozeß ihrer Anreicherung und Lagerung aktiviert werden, Inhaltsstoffe sezernieren und aggregieren. Verschiedene solcher Strategien sind in der Literatur beschrieben; diese reichen von bestimmten Waschprozeduren bis zur Zugabe spezifischer Inhibitoren:
- Der Aktivator der Plättchenaggregation Calcium kann durch ein chelierendes Agenz, z.B. EDTA, komplexiert werden.
- Der Aktivator ADP kann durch Zusatz des Enzyms Apyrase vollständig zu AMP abgebaut werden.
- Plasmafaktoren, die zur Plättchenaktivierung beitragen könnten, können durch Waschen der Plättchen entfernt werden.
- Thrombin kann durch Zugabe von Hirudin oder Heparin/Antithrombin III inhibiert werden.
- Zugabe von Prostacyclin (PGl₂) verhindert die Plättchenaggregation über eine Stimulierung der zellulären Adenylatcyclase.
- Aspirin oder Indomethacin inhibieren die Cyclooxygenase der Plättchen und schalten damit den Thromboxan-Syntheseweg irreversibel aus.

In der Praxis hat sich allerdings gezeigt, daß durch die Zugabe der Inhibitoren die Plättchen irreversibel geschädigt oder stark in ihrer Funktion gehemmt werden. WO-A-9323997 offenbart durch Fixierung und Gefriertrocknung stabilisierte Plättchen. Je nach Fixierung und Aktivator sind jedoch zwischen 25 und 85 % der Plättchen nicht aggregierbar. Ein Verfahren zur schonenden Stabilisierung dieser Zellen ohne wesentliche Beeinträchtigung ihrer Funktion ist wünschenswert.

Jedes Stabilisierungsverfahren muß gewährleisten, daß die Plättchen über den gesamten Prozeß eine gewisse funktionelle Aktivität behalten, dazu gehört zum Beispiel, daß die Plättchen während der Herstellung und Stabilisierung des Konzentrats nicht bereits ihre Form ändern, Aktivatoren ausschütten oder aggregieren. In einer stabilen Plättchenpräparation sollen die Plättchen als Einzelzellen mit überwiegend diskoider Form vorliegen. Die funktionelle Aktivität setzt den Erhalt bestimmter Zellorganellen (z.B. a-Granula) sowie auf molekularer Ebene den Erhalt bestimmter Rezeptoren an der Zelloberfläche, z.B. des Glykoproteins Ib/IX, das als Rezeptor für den von-Willebrand-Faktor dient, oder des Glykoproteins IIb/IIIa, das als Rezeptor für Fibrinogen dient, voraus. Weiterhin ist es notwendig, daß bestimmte Stoffwechselwege intakt bleiben, die in Reaktion auf Bindung von Liganden an die Rezeptoren intrazelluläre Botenstoffe freisetzen und physiologische Prozesse, etwa die Ausschüttung von a-Granula, in Gang setzen.

Beschrieben wurden schon durch Gefriertrocknung stabilisierte Plättchen, die allerdings lediglich auf die Aktivierung durch von-Willebrand-Faktor entsprechend reagieren.

Der vorliegenden Erfindung lag also die Aufgabe zugrunde, eine Plättchenpräparation zur Verfügung zu stellen, die den oben beschriebenen Anforderungen entspricht.

Überraschenderweise wurde gefunden, daß nach Zugabe von Inhibitoren der Plättchenfunktion sowie nachfolgender Gefriertrocknung einzeln vorliegende, funktionell aktive Plättchen erhalten werden konnten.

Funktionell aktiv im Sinne der vorliegenden Erfindung heißt, daß die stabiliserten und rekonstituierten Plättchen mindestens auf die Zugabe jeder der folgenden Substanzen mit einer Ausschüttung plättcheneigener Stoffe, Änderung ihrer geometrischen Form, Agglutination oder Aggregation reagieren.

ADP, Calcium, Kollagen, Arachidonsäure, Thrombin, Antikörper gegen Plättchenbestandteile, Plättchenaktivatoren aus der Gerinnungskaskade. Bevorzugt sind auch Plättchen, die speziell auf einzelne dieser Substanzen oder ausgewählte Kombinationen davon reagieren. Besonders bevorzugt sind Plättchen, die auf Zugabe von Heparin reagieren.

Somit betrifft die Erfindung ein Verfahren zur Gewinnung funktionell aktiver Plättchen, bei dem zunächst Blut entnommen und mit einem Antikoagulanz vermischt wird. Hierbei kommen allgemein übliche Antikoagulanzien wie Citrat oder EDTA in der üblicherweise verwendeten Konzentration Frage. In das Entnahmemedium können weitere Inhibitoren eingeschlossen werden. Vorteilhaft ist zum Beispiel der Einschluß von Thrombininhibitoren in einer Konzentration, die gewährleistet, daß das gesamte Thrombin, das im Blut gebildet werden kann, sicher inhibiert wird. Besonders vorteilhaft ist die Verwendung von Hirudin in einer Endkonzentration von 1 Einheit/ml bis 10 Einheiten/ml von. Als Inhibitor der Plättchenfunktion eignet sich überraschenderweise neben den oben besprochenen spezifischen Inhibitoren der Plättchenfunktion die Substanz Hydroxychlorochinsulfat. Chloroquin und Hydroxychloroquin sind als Malariamittel bekannt. Es handelt sich um kationische amphiphile Drogen, die voll membrangängig sind. Zur Stabilisierung der Plättchen eignen sich millimolare Konzentrationen an Hydroxychloroquinsulfat, vorzugsweise 0,1 bis 5 g/L, besonders bevorzugterweise 5 g/L. Weitere Ausführungsförmen der Erfindung sind den Patentansprüchen 2-17 zu entnehmen.

Aus dem antikoagulierten Blut werden die Plättchen, nach dem Fachmann als solche bekannte Verfahren, durch sequentielle Zentrifugation abgetrennt. In einem ersten Schritt wird z.B. eine Zentrifugation mit 3000 x g für 45 Minuten durchgeführt; Plättchen werden durch Abtrennung des 'Buffy Coat' gewonnen und in einer gepufferten Lösung von Antikoagulanz aufgenommen. Dieses Material wird zur Abtrennung anderer Blutzellen 20 Minuten lang mit 200 x g zentrifugiert; den Überstand bilden Plättchen.

Diese Plättchen werden nun mehrmals mit einem Überschuß Waschpuffer gewaschen. Der Waschpuffer enthält Antikoagulanzien, Puffersubstanzen und Stabilisatoren. Als Antikoagulanz kommen etwa EDTA oder Citrat in Frage. Die Pufferung kann ebenfalls durch Citrat oder andere Puffersysteme (HEPES, Phosphat) erfolgen. Ein Waschpuffer kann bevorzugterweise die folgende Zusammensetzung haben: 32.2 g/L Natriumcitrat, 5 g/L Hydroxychloroquinsulfat, pH 7.4.

Zu der Plättchensuspension wird anschließend noch ein Kuchenbildner für die Lyophilisation zugefügt. Vorteilhaft ist z.B. ein Polysaccharid wie etwa Mannit oder ein Protein, z.B. Polygelin oder Serumalbumin. Bevorzugterweise wird Serumalbumin in einer Endkonzentration von 0,1 bis 100 g/L, ganz bevorzugterweise 10 bis 70 g/L, besonders bevorzugterweise 50 g/L eingesetzt.

Schließlich werden die Plättchen werden auf eine Konzentration zwischen 10⁴/µL und 10⁸/µL, vorzugsweise auf 10⁷/µL eingestellt.

Die Plättchen werden vorteilhafterweise 5 bis 60, bevorzugterweise 10 bis 40, ganz bevorzugterweise etwa 30 Minuten bei Raumtemperatur (10 bis 40, bevorzugterweise 20 bis 25 °C) inkubiert und anschließend so gefriergetrocknet, daß sich eine Restfeuchte zwischen 0 % und 10 %, vorzugsweise etwa 3% einstellt.

Besonders vorteilhaft ist der kombinierte Zusatz eines Inhibitors und eines Kuchenbildners. Dadurch werden offensichtlich die Plättchen soweit stabilisiert, daß sie nach Einfrieren und Gefriertrocknen durch physiologische Aktivatoren aktivierbar sind.

Zur Rekonstitution wird das gefriergetrocknete Plättchenkonzentrat vorteilhafterweise in Aktivierungspuffer rekonstituiert. Dieser enthält z.B.:
- Glucose in einer Konzentration zwischen 0 g/L und 100 g/L, vorzugsweise 1 g/L bis 10 g/L, besonders bevorzugterweise etwa 2.4 g/L,
- ein Magnesiumsalz, vorzugsweise Magnesiumchlorid, in einer Konzentration zwischen 0 g/L und 100 g/L, vorzugsweise 1 g/L bis 5 g/L, besonders bevorzugterweise etwa 1.2 g/L,
- ein Kaliumsalz, vorzugsweise Kaliumchlorid, in einer Konzentration zwischen 0 g/L und 100 g/L, vorzugsweise 1 g/L bis 5 g/L, besonders bevorzugterweise etwa 1.6 g/L
- ein Natriumsalz, vorzugsweise Natriumchlorid in einer Konzentration zwischen 0 g/L und 100 g/L, vorzugsweise 0.5 g/L bis 5 g/L, besonders bevorzugterweise etwa 0.6 g/L.

Um Inhibitoren der Plättchenfunktion zu entfernen, können diese auch in dem oben genannten Aktivierungspuffer z.B. durch Aufnehmen und Abzentrifugieren mit etwa 2400 x g gewaschen werden.

Nach diesen Schritten kann das Plättchenkonzentrat als Standardmaterial für Plättchenfunktionsteste oder als Reagenz in diagnostischen Testen eingesetzt werden.

Die gefriergetrockneten Plättchen sind bei +4 °C mindestens 6 Monate haltbar. Durch die Lagerung wird die aggregometrisch gemessene Reaktivität der Plättchen auf Aktivatoren, z. B. Kollagen oder Thrombin, nicht beeinträchtigt. Gegenüber der Haltbarkeit flüssiger Plättchenkonzentrate (7 Tage) ergibt sich also eine Verbesserung der Stabilität um mindestens den Faktor 30.

Gefriergetrocknete Plättchen können ebenfalls als Medikament zur Behandlung von Plättchenmängeln oder Plättchen-Fehlfunktionen eingesetzt werden.

Zur Herstellung einer therapeutisch nutzbaren Komposition müssen die Plättchen in geeigneter Form formuliert werden. Hierzu dienen dem Fachmann an sich bekannte pharmazeutisch verwendbare Trägersysteme. Vorzugsweise werden die Plättchen als sterile Suspension intravenös verabreicht. Das gefriergetrocknete Material enthält aktivierbare Plättchen als therapeutisch aktives Material neben Hydroxychloroquinsulfat und Serumalbumin als Stabilisatoren. Durch Resuspendierung in sterilem *Aqua ad injectabile* entsteht bereits ein pharmazeutisch direkt einsetzbares Medikament. Sowohl für Hydroxychloroquinsulfat als auch für Serumalbumin liegen umfangreiche Erfahrungen bei der Verwendung beim Menschen vor. Hydroxychloroquinsulfat ist ein altbekanntes Mittel bei der Malariatherapie (Webster (1990) in: The Pharmacological Basis of of Therapeutics; Eds. L.S. Goodman and A. Gilman, 8th Edtn.). Die Einsatzroute ist in der Regel oral, eine intravenöse Anwendung ist aber ebenfalls möglich (White (1988) Eur.J.Clin.Pharm. 34(1), S1-14). Bei der vorzugsweise verwendeten Konzentration von Hydroxychloroquinsulfat von etwa 5 g/L und bei einer Plättchenkonzentration von 10⁷/µL würde die Infusion der normalerweise in einem Liter Blut enthaltenen Plättchenzahl mit der Aufnahme von 250 mg Hydroxychloroquinsulfat verbunden sein. Unter Malariatherapie wird bis zu 500 mg der Substanz pro Tag verwendet. Eine intravenöse Gabe von 0.8 mg pro kg und Stunde ist toxikologisch unbedenklich (White 1988). Es kann also davon ausgegangen werden, daß der Zusatz von Hydroxychloroquinsulfat zu den Plättchen toxikologisch unbedenklich ist.

Humanserumalbumin ist ein bekannter und toxikologisch unbedenklicher Stabilisator, der in vielen zugelassenen Arzneimitteln enthalten ist.

Neben der Infusion der gefriergetrockneten Plättchen unmittelbar nach Einlösen in sterilem *Aqua ad injectabile* sind auch andere Formulierungen der Plättchen zur Verwendung am Patienten möglich. So kann etwa das Hydroxychloroquinsulfat durch, ggf. mehrmaliges, Waschen der zunächst in Wasser resuspendierten Plättchen entfernt werden. Hierzu sind die Plättchen mit etwa 2400 x g abzuzentrifugieren und anschließend in einem pharmazeutisch geeigneten Medium aufzunehmen. Als solche Medien kommen grundsätzlich alle dem Fachmann dafür an sich bekannten Lösungen, wie z.B. sterile Salzlösungen (etwa isotonische NaCl oder andere), sterile Pufferlösungen (Citrat, Tris, HEPES) und sterile Lösungen von Stabilisatoren (Proteine wie Humanserumalbumin oder andere, Zucker wie Mannit oder andere) in Frage.

Die folgenden Beispiele sollen die Erfindung erläutern.

### Beispiele

### Beispiel 1.1: Herstellung eines Plättchenkonzentrats aus Citratblut

Blut gesunder Spender wird durch Venenpunktion entnommen und direkt mit Antikoagulanz vermischt. Hierbei werden 500 mL Blut unter Vermeidung von Schaumbildung in eine Flasche gefüllt, die als Vorlage 50 mL konzentrierten Citratpuffer (39.6 g/L Natriumcitrat + 0.26 g/L Zitronensäure, pH 7) enthält. Zur Durchmischung wird die Flasche vorsichtig bewegt. Das Blut wird in einen Edelstahlbecher überführt und 45 Minuten lang mit ca. 3000 x g bei 12°C - 18°C zentrifugiert. Das überstehende Plasma wird verworfen. Die sich nach unten anschließende zelluläre Schicht ('Buffy Coat') wird in Waschpuffer (32.2 g/L Natriumcitrat, 5 g/L Hydroxychloroquinsulfat, pH 7.4) aufgenommen und zur Abtrennung von Leukozyten zwei Mal für 20 Minuten mit 200 x g zentrifugiert. Der Überstand bildet eine Thrombozytensuspension. Abschließend werden die Zellen noch zwei Mal mit Waschpuffer gewaschen. Hierzu wird die Suspension zunächst 20 Minuten lang mit 2400 x g abzentrifugiert und sodann jeweils in Waschpuffer resuspendiert.

### Beispiel 1.2: Gefriertrocknung eines Plättchenkonzentrats

Eine Plättchensuspension wird gemäß Beispiel 1 hergestellt.

Die Plättchendichte wird mit Hilfe eines automatischen Zählgeräts (Mölab, Hilden, Deutschland) bestimmt. Anschließend wird die Suspension mit Waschpuffer auf 5 x 10⁷/µL eingestellt.

Pro mL der Suspension werden 50 mg Serumalbumin vom Rind zugefügt. Anschließend wird die Suspension abgefüllt und schonend gefriergetrocknet. Die höchste eingestellte Temperatur beträgt 40 °C, die Gesamtdauer der Trocknung ist 25 Stunden. Der Prozeß wird so geführt, daß sich eine Restfeuchte von ca. 1-2 % einstellt.

### Beispiel 2: Aggregation von Plättchen nach Gefriergetrocknung mit Kollagen

Plättchen wurden gemäß Beispiel 1 präpariert und gefriergetrocknet. Das Lyophilisat wird im Ursprungsvolumen Aqua dest. rekonstituiert.
Die Plättchen werden durch Zentrifugation (10 Minuten mit 2500 x g) abzentrifugiert und im gleichen Volumen isotonischer Kochsalzlösung aufgenommen und in dieser Lösung zwei Mal gewaschen. Anschließend werden die Plättchen in Aktivierungspuffer (2.4 g/L Glucose, 0.6 g/L NaCl, 1.2 g/L MgCl2, 1.6 g/L KCl) aufgenommen und mit diesem Puffer auf eine Konzentration von 4.6x10⁵/µL eingestellt. Die Plättchenzählung wird mit einem automatischen Zählgerät (Fa. Mölab, Hilden, Deutschland) durchgeführt .

Als *Positivkontrolle* dient plättchenreiches Citratplasma (Pool gesunder Spender), das mit plättchenarmem Plasma auf 4.8x10⁵/µL eingestellt wird. Als Ne*gativkontrolle* dienen Plättchen, die in 4 % Formalin fixiert und anschließend lyophilisiert wurden. Diese Plättchen werden in isotonischer NaCI-Lösung rekonstituiert und auf 4.9x10⁵/µL eingestellt.

1000 µL Plättchen werden entweder mit 100 µl Kollagen aus menschlicher Plazenta (1 mg/mL, Behring Diagnostics, Marburg, Deutschland) oder mit 100 µl isotonischer NaCl als Kontrolle vermischt und 15 Minuten bei 37°C inkubiert. Anschließend wird die Mischung zur Abtrennung von Aggregaten 10 Minuten lang mit 40 x g zentrifugiert.

Die Plättchendichte im Überstand wurde jeweils bestimmt und ist ein Maß für den Anteil nichtaggregierbarer Plättchen.

| Aktivator | NaCl (Negativkontrolle) | Kollagen |
|---|---|---|
| Plättchenreiches Plasma (Positivkontrolle) | 85 | 9 |
| Fixierte Plättchen (Negativkontrolle) | 77 | 74 |
| Stabilisierte und lyophilisierte Plättchen | 70 | 3 |

### Tabelle 1: Plättchendichte im Überstand

Es zeigt sich, daß unter den gewählten Bedingungen die Plättchen aus plättchenreichem Plasma sowie die lyophilisierten Plättchen nahezu vollständig in Form von abtrennbaren Aggregaten vorliegen, wohingegen die formalinfixierten Plättchen nicht aggregierbar sind.

### Beispiel 3: Aggregation von Plättchen nach Gefriergetrocknung mit Thrombin

Plättchen wurden gemäß Beispiel 1 präpariert und gefriergetrocknet. Das Lyophilisat wird im Ursprungsvolumen Aqua dest. rekonstituiert.

Die Plättchen werden durch Zentrifugation (10 Minuten mit 2500 x g) abzentrifugiert und im gleichen Volumen isotonischer Kochsalzlösung aufgenommen und in dieser Lösung zwei Mal gewaschen. Anschließend werden die Plättchen in Aktivierungspuffer (2.4 g/L Glucose, 0.6 g/L NaCl, 1.2 g/L MgCl2, 1.6 g/L KCI) aufgenommen und mit diesem Puffer auf eine Konzentration von 4.6x10⁵/µL eingestellt. Die Plättchenzählung wird mit einem automatischen Zählgerät (Fa. Mölab, Hilden, Deutschland) durchgeführt.

Als *Negativkontrolle* dienen Plättchen, die in 4 % Formalin fixiert und anschließend lyophilisiert wurden. Diese Plättchen werden in isotonischer NaCl-Lösung rekonstituiert und auf 4.7 x 10⁵/µL eingestellt.

1000 µL Plättchen werden 5 Minuten bei 37°C vorinkubiert. Anschließend wird aufgereinigtes Thrombin vom Rind (100 µL 3 IU/mL; Behring Diagnostics) oder 100 µL isotonische NaCl zugegeben. Es wird 15 Minuten bei 37°C inkubiert. Anschließend wird die Mischung zur Abtrennung von Aggregaten 10 Minuten lang mit 40 x g zentrifugiert.

Die Plättchendichte im Überstand wurde jeweils bestimmt und ist ein Maß für den Anteil nichtaggregierbarer Plättchen.

**Tabelle 2: Plättchendichte im Überstand**

| Aktivator | NaCl (Negativkontrolle) | Thrombin |
|---|---|---|
| Fixierte Plättchen (Negativkontrolle) | 64 | 64 |
| Stabilisierte und lyophilisierte Plättchen | 65 | 19 |

Es zeigt sich, daß unter den gewählten Bedingungen die lyophilisierten Plättchen zu etwa zwei Dritteln in Form von abtrennbaren Aggregaten vorliegen, wohingegen die formalinfixierten Plättchen nicht aggregierbar sind.

## Patentansprüche

1. Durch Gefriertrocknung stabilisierte, funktionell aktive Plättchen, **dadurch gekennzeichnet, daß** diese durch Zugabe von Inhibitoren der Plättchenfunktion sowie nachfolgender Gefriertrocknung erhalten wurden.

2. Plättchen gemäß Anspruch 1, **dadurch gekennzeichnet, daß** sie aus dem Blut von Säugetieren isoliert werden.

3. Plättchen gemäß Anspruch 2, **dadurch gekennzeichnet, daß** das entnommene Blut mit einem Entnahmemedium vermischt wird, wobei das Entnahmemedium ein Antikoagulanz enthält.

4. Plättchen gemäß Anspruch 3, **dadurch gekennzeichnet, daß** dem Entnahmemedium mindestens ein Inhibitor der Plättchenaggregation zugesetzt wird.

5. Plättchen gemäß Anspruch 4, **dadurch gekennzeichnet, daß** das Entnahmemedium Hirudin in einer Endkonzentration zwischen 0.1 E/mL und 10 E/mL, vorzugsweise 1 E/mL bis 4 E/mL, besonders bevorzugterweise etwa 2 E/mL, enthält.

6. Plättchen gemäß Anspruch 4, **dadurch gekennzeichnet, daß** das Entnahmemedium eine der Substanzen Chloroquin, Hydroxychloroquin, Camoquin, Quinacrin und Procain enthält.

7. Plättchen gemäß Anspruch 6, **dadurch gekennzeichnet, daß** Hydroxychloroquin in einer Konzentration zwischen 0.1 g/L und 100 g/L, vorzugsweise 1 g/L bis 10 g/L, besonders bevorzugterweise etwa 5 g/L, verwendet wird.

8. Plättchen gemäß Anspruch 1, **dadurch gekennzeichnet, daß** sie vor der Gefriertrocknung gewaschen werden.

9. Plättchen gemäß Anspruch 8, **dadurch gekennzeichnet, daß** der Waschpuffer Antikoagulanz, vorzugsweise Citrat, in einer Konzentration zwischen 1 g/L und 50 g/L, vorzugsweise 2 g/L bis 10 g/L, besonders bevorzugterweise etwa 3.8 g/L enthält.

10. Plättchen gemäß Anspruch 8, **dadurch gekennzeichnet daß** der Waschpuffer einen Inhibitor aus der Gruppe der folgenden Substanzen enthält: Chloroquin, Hydroxychloroquin, Camoquin, Quinacrin und Procain.

11. Plättchen gemäß Anspruch 10, **dadurch gekennzeichnet, daß** Hydroxychloroquin in einer Konzentration zwischen 0.1 g/L und 100 g/L, vorzugsweise 1g/L bis 10 g/L, besonders bevorzugterweise bis etwa 5 g/L, verwendet wird.

12. Plättchen gemäß Anspruch 1. **dadurch gekennzeichnet, daß** ihnen vor der Gefriertrocknung mindestens ein Stabilisator zugesetzt wird.

13. Plättchen gemäß Anspruch 12, **dadurch gekennzeichnet, daß** als Stabilisator einer der folgenden Stoffe zugesetzt wird: Serumalbumin in einer Konzentration zwischen 1 g/L und 100 g/L, vorzugsweise 20 g/L bis 60 g/L, besonders bevorzugterweise etwa 50 g/L, ein Kohlenhydrat, vorzugsweise Mannit, in einer Konzentration zwischen 1 mg/L und 100 g/L, vorzugsweise 5 g/L bis 30 g/L, besonders bevorzugterweise etwa 10 g/L, Polygelin in einer Konzentration zwischen 1 mg/L und 100 g/L, vorzugsweise 5 g/L bis 30 g/L, besonders bevorzugterweise 10 g/L.

14. Plättchen gemäß Anspruch 1, **dadurch gekennzeichnet, daß** sie vor der Gefriertrocknung auf eine Konzentration zwischen 10⁴/µL und 10⁹/µL eingestellt werden.

15. Plättchen gemäß Anspruch 1, **dadurch gekennzeichnet, daß** die Gefriertrocknung so durchgeführt wird, daß sich eine Restfeuchte zwischen 0 % und 10 %, vorzugsweise etwa 3 % einstellt.

16. Plättchen gemäß Anspruch 1, **dadurch gekennzeichnet, daß** sie nach der Gefriertrocknung in Aktivierungspuffer rekonstituiert werden.

17. Plättchen gemäß Anspruch 16, **dadurch gekennzeichnet, daß** der Aktivierungspuffer folgende Stoffe enthält: Glucose in einer Konzentration zwischen 1 mg/L und 100 g/L, vorzugsweise 0.1g/L bis 5 g/L, besonders bevorzugterweise etwa 2.4 g/L, ferner ein Magnesiumsalz, vorzugsweise Magnesiumchlorid, in einer Konzentration zwischen 1 mg/L und 100 g/L, vorzugsweise 0.1 g/L bis 5 g/L, besonders bevorzugterweise etwa 1.2 g/L, ferner ein Kaliumsalz, vorzugsweise Kaliumchlorid, in einer Konzentration zwischen 1 mg/L und 100 g/L, vorzugsweise etwa 0.1 g/L bis 5 g/L, besonders bevorzugterweise etwa 1.6 g/L und ein Natriumsalz, vorzugsweise Natriumchlorid in einer Konzentration zwischen 1 mg/L und 100 g/L, vorzugsweise 0.1 g/L bis 5 g/L, besonders bevorzugterweise etwa 0.6 g/L.

18. Reagenz, **dadurch gekennzeichnet, daß** es Plättchen gemäß Anspruch 1 enthält.

19. Reagenz gemäß Anspruch 18, **dadurch gekennzeichnet, daß** es als Standard oder Kontrollmaterial für Plättchenfunktionsteste dient.

20. Reagenz gemäß Anspruch 18, **dadurch gekennzeichnet, daß** es zum Nachweis der heparininduzierten Thrombozytopenie eingesetzt wird.

21. Pharmazeutische Komposition zur Behandlung von unzureichender Plättchenfunktion beim Patienten, **dadurch gekennzeichnet, daß** es eine therapeutische Menge von Plättchen gemäß Anspruch 1 und ein pharmazeutisch akzeptables Trägermaterial enthält.

## Claims

1. Freeze drying-stabilized, functionally active platelets which were obtained by adding inhibitors of platelet function and of subsequent freeze drying.

2. The platelets as claimed in claim 1, which are isolated from the blood of mammals.

3. The platelets as claimed in claim 2, wherein the blood which is withdrawn is mixed with a withdrawal medium, with the withdrawal medium comprising an anticoagulant.

4. The platelets as claimed in claim 3, wherein at least one inhibitor of platelet aggregation is added to the withdrawal medium.

5. The platelets as claimed in claim 4, wherein the withdrawal medium comprises hirudin at a final concentration of between 0.1 U/ml and 10 U/ml, preferably of from 1 U/ml to 4 U/ml, particularly preferably of about 2 U/ml.

6. The platelets as claimed in claim 4, wherein the withdrawal medium comprises one of the substances chloroquine, hydroxychloroquine, camoquin, quinacrine and procaine.

7. The platelets as claimed in claim 6, wherein hydroxychloroquine is used at a concentration of between 0.1 g/l and 100 g/l, preferably of from 1 g/l to 10 g/l, particularly preferably of about 5 g/l.

8. The platelets as claimed in claim 1, which are washed before the freeze-drying.

9. The platelets as claimed in claim 8, wherein the washing buffer comprises an anticoagulant, preferably citrate, at a concentration of between 1 g/l and 50 g/l, preferably of from 2 g/l to 10 g/l, particularly preferably of about 3.8 g/l.

10. The platelets as claimed in claim 8, wherein the washing buffer comprises an inhibitor from the group of the following substances: chloroquine, hydroxychloroquine, camoquin, quinacrine and procaine.

11. The platelets as claimed in claim 10, wherein hydroxychloroquine is used at a concentration, of between 0.1 g/l and 100 g/l, preferably of from 1 g/l to 10 g/l, particularly preferably of up to about 5 g/l.

12. The platelets as claimed in claim 1, wherein at least one stabilizer is added to them before the freeze-drying.

13. The platelets as claimed in claim 12, wherein one of the following substances is added as a stabilizer; serum albumin at a concentration of between 1 g/l and 100 g/l, preferably of from 20 g/l to 60 g/l, particularly preferably of about 50 g/l, a carbohydrate, preferably mannitol, at a concentration of between 1 mg/l and 100 g/l, preferably of from 5 g/l to 30 g/l, particularly preferably of about 10 g/l, or polygeline at a concentration of between 1 mg/l and 100 g/l, preferably of from 5 g/l to 30 g/l, particularly preferably of 10 g/l.

14. The platelets as claimed in claim 1, which are adjusted before the freeze-drying to a concentration of between 10⁴/µl and 10⁹/µl.

15. The platelets as claimed in claim 1, wherein the freeze-drying is carried out such that a residual moisture content of between 0% and 10%, preferably of about 3%, is obtained.

16. The platelets as claimed in claim 1, which are reconstituted in activation buffer after the freeze-drying.

17. The platelets as claimed in claim 16, wherein the activation buffer comprises the following substances: glucose at a concentration of between 1 mg/l and 100 g/l, preferably of from 0.1 g/l to 5 g/l, particularly preferably of about 2.4 g/l, and, in addition, a magnesium salt, preferably magnesium chloride, at a concentration of between 1 mg/l and 100 g/l, preferably of from 0.1 g/l to 5 g/l, particularly preferably of about 1.2 g/l, and, in addition, a potassium salt, preferably potassium chloride, at a concentration of between 1 mg/l and 100 g/l; preferably of from about 0.1 g/l to 5 g/l, particularly preferably of about 1.6 g/l, and a sodium salt, preferably sodium chloride, at a concentration of between 1 mg/l and 100 g/l, preferably of from 0.1 g/l to 5 g/l, particularly preferably of about 0.6 g/l.

18. A reagent which comprises platelets as claimed in claim 1.

19. The reagent as claimed in claim 18, which is used as standard or control material for platelet function tests.

20. The reagent as claimed in claim 18, which is employed for detecting heparin-induced thrombocytopenia.

21. A pharmaceutical composition for treating inadequate platelet function in a patient, which comprises a therapeutic quantity of platelets as claimed in claim 1 and a pharmaceutically acceptable carrier material.

## Revendications

1. Plaquettes fonctionnellement actives, stabilisées par lyophilisation, **caractérisées en ce qu'**elles sont obtenues par une addition d'inhibiteurs de la fonction plaquettaire et une lyophilisation ultérieure.

2. Plaquettes selon la revendication 1, **caractérisées en ce qu'**elles sont isolées à partir du sang de mammifères.

3. Plaquettes selon la revendication 2, **caractérisées en ce que** le sang prélevé est mélangé avec un milieu de prélèvement, ledit milieu de prélèvement contenant un anticoagulant.

4. Plaquettes selon la revendication 3, **caractérisées en ce qu'**on ajoute au milieu de prélèvement au moins un inhibiteur d'agrégation plaquettaire.

5. Plaquettes selon la revendication 4, **caractérisées en ce que** le milieu de prélèvement contient de l'hirudine avec une concentration finale comprise entre 0,1 U/ml et 10 U/ml, de préférence de 1 U/ml à 4 U/ml, de façon particulièrement préférée d'environ 2 U/ml.

6. Plaquettes selon la revendication 4, **caractérisées en ce que** le milieu de prélèvement contient l'une des substances chloroquine, hydroxychloroquine, camoquine, quinacrine et procaïne.

7. Plaquettes selon la revendication 6, **caractérisées en ce que** l'hydroxychloroquine est utilisée à une concentration comprise entre 0,1 g/l et 100 g/l, de préférence de 1 g/l à 10 g/l, de façon particulièrement préférée d'environ 5 g/l.

8. Plaquettes selon la revendication 1, **caractérisées en ce qu'**elles sont lavées avant la lyophilisation.

9. Plaquettes selon la revendication 8, **caractérisées en ce que** le tampon de lavage contient un anticoagulant, de préférence un citrate, à une concentration comprise entre 1 g/l et 50 g/l, de préférence de 2 g/l à 10 g/l, de façon particulièrement préférée d'environ 3,8 g/l.

10. Plaquettes selon la revendication 8, **caractérisées en ce que** le tampon de lavage contient un inhibiteur choisi dans le groupe des substances suivantes : chloroquine, hydroxychloroquine, camoquine, quinacrine et procaïne.

11. Plaquettes selon la revendication 10, **caractérisées en ce que** l'hydroxychloroquine est utilisée à une concentration comprise entre 0,1 g/l et 100 g/l, de préférence de 1 g/l à 10 g/l, de façon particulièrement préférée d'environ 5 g/l.

12. Plaquettes selon la revendication 1, **caractérisées en ce qu'**on y ajoute au moins un stabilisant avant la lyophilisation.

13. Plaquettes selon la revendication 12, **caractérisées en ce qu'**on ajoute comme stabilisant l'une des substances suivantes : de l'albumine-sérique à une concentration comprise entre 1 g/l et 100 g/l, de préférence de 20 g/l à 60 g/l, de façon particulièrement préférée d'environ 50 g/l, un glucide, de préférence le mannitol, à une concentration comprise entre 1 mg/l et 100 g/l, de préférence de 5 g/l à 30 g/l, de façon particulièrement préférée d'environ 10 g/l, de la polygéline à une concentration comprise entre 1 mg/l et 100 g/l, de préférence de 5 g/l à 30 g/l, de façon particulièrement préférée de 10 g/l.

14. Plaquettes selon la revendication 1, **caractérisées en ce qu'**elles sont ajustées, avant la lyophilisation, à une concentration comprise entre 10⁴/µl et 10⁹/µl.

15. Plaquettes selon la revendication 1, **caractérisées en ce que** la lyophilisation est effectuée de manière que s'ajuste une humidité résiduelle comprise entre 0 % et 10 %, de préférence d'environ 3 %.

16. Plaquettes selon la revendication 1, **caractérisées en ce qu'**elles sont reconstituées, après la lyophilisation, dans un tampon d'activation.

17. Plaquettes selon la revendication 16, **caractérisées en ce que** le tampon d'activation contient les substances suivantes : du glucose à une concentration comprise entre 1 mg/l et 100 g/l, de préférence de 0,1 g/l à 5 g/l, de façon particulièrement préférée d'environ 2,4 g/l, en outre, un sel de magnésium, de préférence le chlorure de magnésium, à une concentration comprise entre 1 mg/l et 100 g/l, de préférence de 0,1 g/l à 5 g/l, de façon particulièrement préférée d'environ 1,2 g/l, en outre un sel de potassium, de préférence le chlorure de potassium, à une concentration comprise entre 1 mg/l et 100 g/l, de préférence d'environ 0,1 g/l à 5 g/l, de façon particulièrement préférée d'environ 1,6 g/l, et un sel de sodium, de préférence le chlorure de sodium, à une concentration comprise entre 1 mg/l et 100 g/l, de préférence de 0,1 g/l à 5 g /l, de façon particulièrement préférée d'environ 0,6 g/l.

18. Réactif, **caractérisé en ce qu'**il contient des plaquettes selon la revendication 1.

19. Réactif selon la revendication 18, **caractérisé en ce qu'**il sert de substance de référence ou de substance témoin pour des tests de fonction des plaquettes.

20. Réactif selon la revendication 18, **caractérisé en ce qu'**il est utilisé pour la détection de la thrombopénie induite par l'héparine.

21. Composition pharmaceutique pour le traitement d'une insuffisance de la fonction plaquettaire chez un patient, **caractérisée en ce qu'**elle contient une quantité thérapeutique de plaquettes selon la revendication 1 et un véhicule pharmaceutiquement acceptable.
